# EUROPEAN PATENT SPECIFICATION

(11) **EP 0 323 831 B1**
(45) Date of publication and mention of the grant of the patent: **13.10.1993**
(21) Application number: 89100053.1
(22) Date of filing: 03.01.1989
(51) Int. Cl.: C07C 39/16, C07C 37/20

(54) **Process for preparing bisphenol A**
Verfahren zur Herstellung von Bisphenol A
Procédé pour la fabrication de bisphénol A

(30) Priority: 08.01.1988 JP 1348/88
(43) Date of publication of application: 12.07.1989
(73) Proprietor: MITSUI TOATSU CHEMICALS, Inc., Chiyoda-Ku Tokyo 100 (JP)
(72) Inventor: Iimuro, Shigeru, Nagoya-shi Aichi (JP); Morimoto, Yoshio, Tokai-shi Aichi (JP); Kitamura, Takashi, Nagoya-shi Aichi (JP)
(74) Representative: Strehl Schübel-Hopf Groening & Partner

(56) References cited:
- DD-A- 148 515
- DE-A- 2 364 164
- DE-B- 1 244 796
- DE-B- 1 518 321
- FR-A- 2 397 383
- GB-A- 785 079
- US-A- 2 730 553

## Description

The present invention relates to a process for preparing high purity 2,2-bis(4-hydroxyphenyl)propane (hereinafter referred to as bisphenol A, p,p′-isomer).

Bisphenol A is used as a raw material for polycarbonate resins or epoxy resins, and colorless and high purity bisphenol A is required for polycarbonate resins in particular.

Bisphenol A is prepared from acetone and excess phenol in the presence of an acidic catalyst, in some cases by the addition of a co-catalyst such as sulfur compounds. The reaction mixture contains bisphenol A, the catalyst, unreacted acetone, unreacted phenol, water and other by-products of the reaction.

The by-products are mainly composed of 2-(2-hydroxyphenyl)-2-(4-hydroxyphenyl)propane (hereinafter referred to as o,p′-isomer) and also contain Dianin's compound, trisphenol, polyphenol and undesirable colored substances These by-products deteriorate the properties of resins prepared from bisphenol A.

Hydrochloric acid or strongly acidic ion exchange resin are known as a catalyst of this reaction. In the use of hydrochloric acid, the adduct of bisphenol A and phenol is precipitated while effecting the reaction in a low temperature. At the same time, o,p′-isomer is isomerized to p,p'-isomer and consequently, the amount of o,p'-isomer can be reduced.

On the other hand, Dianin's compound can be reduced by adding 3 % or more of water by the method described in Japanese Patent Publication No. 40-7186 or by the addition of mercapto-compounds as described in Japanese Patent Publication No. 27-5367. However, adding a large amount of water requires many steps such as dehydration, and the separation and recovery of hydrochloric acid after the reaciton. Adding a mercapto-compound also requires complicated separation steps therefor and results in emitting obnoxious odor. These two methods are, therefore, not put to practical use for industrial purposes.

The amount of Dianin's compound can also be reduced using an excess mol of phenol to acetone. However, the formation of o,p'-isomer increases with decrease of adduct crystals. Bisphenol A must be isolated from phenol present in large quantities in the reaction mixture.

Continuous reaction requires to use an excess phenol to remove a slurry containing the adduct crystals, resulting in increasing the amount of o,p'-isomer. Such a continuous reaction process is disclosed in US-A-2,730,553 and GB-A-785 079, respectively. In the process according to US-A-2,730,553, the acid-catalyzed condensation of carbonyl compounds with an excess of phenol is conducted in a reactor and at least the greater part of the reactor effluent is passed directly into a soaking zone that is operated on a continuous basis at a temperature of from about 20 to about 110°C, wherein the reaction mixture is maintained in the liquid phase and agitated in order to complete the reaction and to obtain a higher yield of bis(hydroxyphenyl)alkanes.

GB-A-785 079 also discloses a process for producing bis-(hydroxyaryl)compounds, in which an excess of phenol is reacted with a ketone in successive reaction stages, wherein both reaction stages are conducted continuously. In both reactions the products are maintained in the liquid state and the o,p'-isomer and the p,p'-isomer are present in the equilibrium state. Thus, there hardly occurs isomerization of the o,p'-isomer to the corresponding p,p'-isomer in the reaction solution and therefore high purity bisphenol A cannot be obtained.

On the other hand, in a batch method, the amount of Dianin's compound (o,p-isomer) increases owing to the high initial concentration of acetone. Such a batch method is disclosed in DE-A-1,518,321. This citation discloses that the isomerization from o,p- to p,p'-bisphenol A occurs when the adduct of bisphenol A with phenol is precipitated and that this precipitation occurs when the reaction mixture is maintained at a temperature below 60°C, which decreases the amount of the o,p-isomer in the final product to a certain extent. However, in this process the problem of an production of the o,p-isomer beside the p,p'-isomer maintains.

In the use of a strongly acidic ion exchange resin, many impurities are produced. However, Dianin's compound is greatly reduced when a part of functional groups of the resin is modified by compounds having a mercapto-group such as mercapto alkyl amine.

In the use of the ion exchange resin catalyst, much more of the o,p′-isomer is produced than when a hydrochloric acid catalyst is used because the isomerization procedure of crystallizing the adduct of phenol and bisphenol A cannot be employed. In addition, in case of the ion exchange resin catalyst, acetone cannot be fully converted owing to water formed in the reaction as described in Japanese Patent Publication Kokai No. 61-78741. A batch method requires dehydration of the resin at each reaction, while a continuous reaction requires an enormous amount of resin for advancing the conversion to some extent. When acetone is isolated and recovered from water, there is no corrosion problem as in the use of hydrochloric acid, however, for isolating acetone from water and recovering the acetone the same facilities and cost are required. The preparation of bisphenol A in phenol having a limited solubility cannot be carried out in high concentration, so that much energy and labor are required for obtaining an end product.

Thus, each of the conventional processes for preparing bisphenol A can reduce the formation of specific impurities but cannot simultaneously reduce the formation of two typical impurities - o,p′-isomer and Dianin's compound to a satisfactory extent.

### SUMMARY OF THE INVENTION

The object of this invention is to provide a process for preparing high purity bisphenol A by decreasing the formation of by-products as much as possible and simplifying the purification treatment as much as possible.

The inventors have diligently investigated in order to achieve the above stated objects and as a result, found that the object of the present invention was achieved by effecting the reaction in two stages of a continuous and batch type.

In accordance with the present invention, there is provided a process for preparing bisphenol A comprising:
a) feeding continuously phenol, acetone, and hydrogen chloride or hydrochloric acid into a reactor maintained at a temperature of 30 to 100°C in a first stage and reacting phenol and acetone to an extent of 20 - 60 mol% of acetone conversion to obtain a first reaction product;
b) removing continuously the first reaction product from the reactor in the first stage;
c) feeding the first reaction product and hydrogen chloride or hydrochloric acid into a reactor which is maintained at a temperature of 30 to 85°C in a second stage which is operated batchwise and completing the reaction of phenol and acetone to obtain a second reaction product as a slurry comprising crystals of the adduct of phenol and bisphenol A;
and d) removing from the second reaction product unreacted phenol, and water and hydrochloric acid and recovering bisphenol A.

### DETAILED DESCRIPTION OF THE INVENTION

According to the process of this invention, 4 - 12 mols of phenol are usually reacted with 1 mol of acetone without substantially using a solvent as the third component. In this case, a small amount of water or hydrochloric acid may be added in order to accelerate the reaction.

The total amount of acetone may be fed into the reactor in the first stage which is operated continuously. Alternatively, a part of the acetone may also be fed into the reactor of batch type in the second stage. The reaction in each stage may be conducted in several reactors which are in series or parallel. In preferable embodiment, reaction products are fed in order from the reactor of continuous type to reactors of batch type.

The reactor may be saturated with hydrogen chloride gas before the reaction. It may also be continuously fed into the reactor during the reaction. It is preferably fed before and after the reaction because there are the heat caused by absorbing hydrogen chloride, the heat of reaction, and the crystallization heat of the adduct. The heat is removed by the external cooling to control the reaction temperature in the prescribed range.

The reaction in the first stage is conducted in a reactor which is operated continuously. This reaction is usually conducted under pressures from normal pressure to 5 bar (kg/cm²) at 30 to 100°C, preferably 40 to 70°C. When the reaction temperature is lower than 30°C, the reaction rate is slow. When it is higher than 100°C, by-products are produced in large quantities. Reaction time depends upon the molar ratio of acetone to phenol, and reaction temperature. The reaction mixture is, preferably, fed to next reactor before the adduct is deposited, that is, before the solubility of the adduct reaches the saturation. The mean retention time required for the saturation is approximately 1.4 hours when the molar ratio of phenol to acetone is 7 and reaction temperature is 40°C, and approximately 1.8 hours when the molar ratio is 6 and reaction temperature is 55°C.

When this adduct has reached the saturation, the crystals are deposited rapidly from the reaction mixture so that the transfer of the reaction slurry becomes difficult and therefore, it is necessary to exactly control the acetone conversion in the reactor in the first stage.

If the acetone conversion in the reactor in the first stage is too low, the initial concentration of acetone in the second reactor is increased and the result is similar to when the total amount of acetone is converted in the reactor of batch type i.e. increase of the Dianin's compound. Also, if the acetone conversion is too high, continuous operation is difficult because the reaction mixture cannot be transferred to next reactor owing to the deposit of the adduct, the adhesion of the crystals to the wall of the reactor, and the growth of the crystals. It is preferred that the acetone conversion is 20 to 60 %.

The molar ration of phenol to acetone is substantially increased by effecting a continuous reaction in the first stage, thus the formation of Dianin's compound being remarkably reduced as compared with the batch reaction in the same molar ratio.

The reaction in the second stage can be conducted by conventional methods except that the reaction mixture in the first stage is fed as the raw material. The reaction is conducted at 30 to 85°C, preferably 35 to 60°C under stirring.

The adduct crystals are deposited as the reaction is advanced. At the same time, the o,p′-isomer in the reaction solution is isomerized to the corresponding p,p′-isomer, thereby decreasing the ratio of o,p′-isomer to bisphenol A in the reaction mixture. Lowering the final reaction temperature to 35 to 45°C permits more effective isomerization. Only a small amount of Dianin's compound is produced even at the time of completion of the reaction in the second stage because the production of Dianin's compound is inhibited in the reactor in the first stage.

Bisphenol A is obtained by removing water, the catalyst and excess phenol from the reaction mixture obtained. This bisphenol A as it is, may be subjected to steps of forming granules, flakes and others to obtain a final product. Alternatively, this bisphenol A may be subjected to a purification procedure, followed by forming steps to obtain a final product. For examples, according to a conventional purification procedure, the adduct is crystalized and thereafter, phenol is removed, for example by distillation to obtain colorless bisphenol A of high purity.

### EXAMPLES

This invention will be hereinafter described in detail with reference to examples and comparative examples. The analysis of acetone is in accordance with potentiometric titration and the analysis of o,p′-isomer and Dianin's compound is effected by gas chromatography.

### Example 1

Acetone (58 kg/h) was added to 564 kg/h of phenol and these were fed continuously into a first reactor controlled at 50°C while blowing continuously 5 kg/h of hydrogen chloride gas into the first reactor. The reaction mixture was continuously removed after 1.5 hours of mean retention time. the acetone conversion was 55 %. 3.2 wt% o,p′-isomer, based on bisphenol A and 0.3 wt% Dianin's compound, based on bisphenol A, were produced. The reaction mixture was fed into a second reactor (internal volume: 1.2 m³) over 1.2 hours. The second reaction was started while blowing 5 kg/h of hydrogen chloride gas into the second reactor and stirring the second reactor, and completed after 7 hours. The reaction temperature was 60°C at maximum and 45°C at the completion of the reaction. When the slurry of this reaction mixture was analyzed, the amounts of o,p′-isomer and Dianin's compound were 1.5 wt% and 0.4 wt% based on bisphenol A, respectively.

### Example 2

Acetone (58 kg/h) was added to 564 kg/h of phenol, and these were fed continuously into the first reactor controlled at 50°C while blowing 5 kg/h of hydrogen chloride gas into the first reactor. The reaction mixture was continuously removed after 0.8 hour of mean retention time. The acetone conversion was 25 %, and 4.5 wt% of the o,p′-isomer and 0.2 wt% of Dianin's compound, each based on bisphenol A, were produced.

The reaction mixture was fed into the second reactor (internal volume: 1.2 m³) over 1.2 hours. The second reaction was started by blowing 5 kg/h of hydrogen chloride gas into the second reactor and stirring the second reactor, and completed after 9 hours. When the slurry of the reaction mixture was analyzed, the amounts of the o,p′-isomer and Dianin's compound were 1.5 wt% and 0.5 wt%, respectively, based on bisphenol A.

### Comparative example 1

Acetone of (58 kg/h) was added to 564 kg/h of phenol and these were fed continuously into the first reactor controlled at 50°C while blowing 5 kg/h of hydrogen chloride gas into the first reactor. The reaction mixture was continuously removed after 2.5 hours of mean retention time. However, the crystals began to grow on the inside wall of the first reactor 4 hours after the operation had started. Because pipes for feeding were clogged after 7 hours, the continuous operation could not be carrried out. The acetone conversion was 65 % until the reaction was stopped. 4.0 wt% of the o,p′-isomer and 0.4 wt% of Dianin's compound, each based on bisphenol A, were produced.

### Comparative example 2

The reaction was carried out in the same manner as in example 1 except that the mixture of phenol and acetone was directly fed into the second reactor without being fed into the first reactor. The amount of hydrogen chloride gas fed was 10 kg/h. The acetone conversion was 99.0 % after 8 hours. 1.6 wt% of the o,p′-isomer and 0.7 wt% of Dianin's compound, based on bisphenol A, were produced. The acetone conversion was 99.5% after 10 hours. The amount of the o,p′-isomer was 1.5 wt%, and that of Dianin's compound was 0.8 wt% based on bisphenol A.

### EFFECT OF THE INVENTION

According to this invention the formation of by-products can be remarkably reduced as compared with the methods using either one conventional reactor operated batchwise or several reactors in series operated continuously. High purity bishenol A can be readily obtained by isolating bisphenol A from the reaction mixture thus obtained, according to the isolation methods known per se.

## Claims

1. A process for preparing bisphenol A comprising:
a) continuously feeding phenol, acetone and hydrogen chloride or hydrochloric acid into a first stage reactor maintained at a temperature of 30 to 100 °C, and reacting phenol with acetone until the acetone conversion is in the range of 20 to 60 mol% to obtain a first reaction product;
b) continuously removing the first reaction product from the first stage reactor;
c) feeding the first reaction product and hydrogen chloride or hydrochloric acid into a second stage reactor maintained at a temperature of 30 to 85 °C, which is operated batchwise and completing the reaction of phenol with acetone to obtain a second reaction product as a slurry comprising crystals of the adduct of phenol and bisphenol A and
d) removing from the second reaction product, unreacted phenol, water and hydrochloric acid, and recovering bisphenol A.

2. The process of claim 1 wherein the first reaction product is continuously removed from the reactor in the first stage before the adduct of bisphenol A and phenol is deposited.

3. The process of claim 1 or 2 wherein the molar ratio of phenol to acetone fed into the reactor in the first stage is 4:1 to 12:1.

4. The process of any of the claims 1 to 3 wherein the reaction in the second stage is conducted using at least two reactors which are operated batchwise.

## Patentansprüche

1. Verfahren zur Herstellung von Bisphenol A, welches umfaßt :
(a) die kontinuierliche Zufuhrung von Phenol, Aceton und Chlorwasserstoff oder Chlorwasserstoffsäure in einen Reaktor der ersten Stufe, der bei einer Temperatur von 30 bis 100°C gehalten wird, und Umsetzen von Phenol mit Aceton, bis eine Umwandlung von Aceton im Bereich von 20 bis 60 Mol.-% erreicht ist, wobei ein erstes Reaktionsprodukt erhalten wird,
(b) kontinuierliches Entnehmen des ersten Reaktionsprodukts aus dem Reaktor der ersten Stufe,
(c) Einführen des ersten Reaktionsprodukts und von Chlorwasserstoff oder Chlorwasserstoffsäure in einen Reaktor der zweiten Stufe, der bei einer Temperatur von 30 bis 85°C gehalten wird und der diskontinuierlich betrieben wird, und Vervollständigen der Umsetzung von Phenol mit Aceton, wobei ein zweites Reaktionsprodukt in Form einer Aufschlämmung gebildet wird, die Kristalle des Addukts von Phenol und Bisphenol A enthält, und
(d) Entnehmen des zweiten Reaktionsprodukts, von nicht umgesetztem Phenol, Wasser und Chlorwasserstoffsäure und Gewinnen von Bisphenol A.

2. Verfahren nach Anspruch 1, bei dem das erste Reaktionsprodukt kontinuierlich aus dem Reaktor der ersten Stufe entnommen wird, bevor das Addukt von Bisphenol A und Phenol abgeschieden wird.

3. Verfahren nach Anspruch 1 oder 2, bei dem das Molverhältnis von dem Reaktor in der ersten Stufe zugeführtem Phenol zu Aceton 4:1 bis 12:1 beträgt.

4. Verfahren nach einem der Ansprüche 1 bis 3, bei dem die Reaktion in der zweiten Stufe unter Verwendung von mindestens zwei diskontinuierlich betriebenen Reaktoren durchgeführt wird.

## Revendications

1. Procédé de préparation de bisphénol A comprenant les étapes suivantes :
a) introduire en continu du phénol, de l'acétone et du chlorure d'hydrogène ou de l'acide chlorhydrique dans un réacteur de première étape maintenu à une température comprise entre 30 et 100°C, et faire réagir le phénol avec l'acétone jusqu'à ce que la conversion de l'acétone soit comprise dans une gamme allant de 20 à 60 % en moles afin d'obtenir un premier produit de réaction ;
b) éliminer en continu le premier produit de réaction du réacteur de première étape ;
c) introduire le premier produit de réaction et du chlorure d'hydrogène ou de l'acide chlorhydrique dans un réacteur de seconde étape maintenu à une température comprise entre 30 et 85°C, qui fonctionne en discontinu et achever la réaction du phénol et de l'acétone afin d'obtenir un second produit de réaction sous forme d'une suspension épaisse contenant des cristaux de l'adduit de phénol et de bisphénol A et
d) éliminer du second produit de réaction le phénol n'ayant pas réagi, l'eau et l'acide chlorhydrique, et récupérer le bisphénol A.

2. Procédé selon la revendication 1, dans lequel le premier produit de réaction est éliminé de façon continue du réacteur au cours de la première étape avant que l'adduit de bisphénol A et de phénol se dépose.

3. Procédé selon la revendication 1 ou 2, dans lequel le rapport molaire du phénol a l'acétone introduits dans le réacteur au cours de la première étape est de 4:1 à 12:1.

4. Procédé selon l'une quelconque des revendications 1 à 3, dans lequel, au cours de la seconde étape, la réaction est effectuée en utilisant au moins deux réacteurs qui fonctionnent en discontinu.
